# EUROPÄISCHE PATENTSCHRIFT

(11) **EP 2 632 508 B1**
(45) Veröffentlichungstag und Bekanntmachung des Hinweises auf die Patenterteilung: **30.09.2015**
(21) Anmeldenummer: 11793652.6
(22) Anmeldetag: 27.10.2011
(51) Int. Cl.: A61M 1/36

(54) **VORRICHTUNG ZUR SELEKTIVEN KÜHLUNG PHYSIOLOGISCHEN GEWEBES**
DEVICE FOR SELECTIVELY COOLING PHYSIOLOGICAL TISSUE
DISPOSITIF DESTINÉ AU REFROIDISSEMENT SÉLECTIF DE TISSU PHYSIOLOGIQUE

(30) Priorität: 27.10.2010 DE 102010049477
(43) Veröffentlichungstag der Anmeldung: 04.09.2013
(73) Patentinhaber: Barbknecht, Ingrid, 36318 Schwalmtal (DE)
(72) Erfinder: Barbknecht, Ingrid, 36318 Schwalmtal (DE)
(74) Vertreter: Quermann, Helmut
(86) Internationale Anmeldenummer: PCT/EP2011/005413
(87) Internationale Veröffentlichungsnummer: WO 2012/055557

(56) Entgegenhaltungen:
- EP-A1- 1 715 279
- WO-A2-2005/117546
- WO-A2-2006/116603

## Beschreibung

Die vorliegende Erfindung betrifft eine Vorrichtung zur selektiven Kühlung des menschlichen oder tierischen Körpers, insbesondere zur extrakorporalen Kühlung einer Körperflüssigkeit, etwa zur Blutkühlung bzw. zur generellen Regulierung der Blut- und Körpertemperatur.

Die Regelung der Körpertemperatur gewinnt in der Medizin eine immer größere Bedeutung. So werden beispielsweise chirurgische Eingriffe am Herzen bei abgesenkter Körpertemperatur durchgeführt. Die Temperatur des Blutes kann hierbei unterhalb 30 °C abgesenkt und nach erfolgter Operation wieder langsam auf Normaltemperatur zurückgeführt werden.

Ferner erweist es sich gerade in Notfallsituationen, so etwa bei Herzstillstand, Schlaganfall sowie Schädel-Hirn-Traumata als besonders vorteilhaft, zumindest das Gehirn und die Gehirnzellen durch sogenannte milde Hypothermie, das heißt durch eine Absenkung der Körper- oder Gehirntemperatur auf Temperaturen zwischen 32 °C und 35 °C abzusenken. So kann einer irreversiblen und massiven Schädigung des Gehirns insbesondere durch eine kontrollierte, in einer Notfallsituation jedoch zügig anzuwendenden Hypothermie effektiv entgegengewirkt werden.

Gerade bei einer drohenden Unterversorgung des Gehirns mit Sauerstoff ist ein zügiges Herbeiführen einer Hypothermie erforderlich. Eine kontrollierte, für medizinische Zwecke geeignete Kühlung des Körpers, insbesondere des Gehirns, ist derzeit nur stationär, nicht aber im ambulanten und mobilen Einsatz durchzuführen. Gängige Methoden zur Körperkühlung basieren zum Beispiel auf der Verwendung eines KühlKatheters, der unter sterilen Bedingungen in ein blutführendes Gefäß eines Patienten einzuführen und mit einer Kühlflüssigkeit beaufschlagt wird. Ein Wärmeaustausch zwischen KühlKatheter und Blut findet hierbei in vivo statt.

Das Einführen des Katheters erweist sich durchaus als problematisch und erfordert speziell geschultes Personal. Zudem werden durch die geometrische Ausdehnung des Katheters typischerweise bis zu 30 und mehr Zentimeter Gefäßlumen eingeengt, sodass der Blutfluss im betreffenden Gefäß erheblich eingeschränkt sein kann.

So ist z.B. aus der WO 2010/111778 A1 eine Vorrichtung zur extrakorporalen Kühlung zum selektiven Kühlen des Gehirns während einer hyper- oder hypothermischen Behandlung eines Patienten bekannt. Die Vorrichtung weist einen Einlass sowie einen Auslass auf, welche beide mit einer Arterie des Patienten zu koppeln sind. Unter Verwendung einer Pumpe wird das über den Einlass bereitgestellte Blut durch einen Wärmetauscher geleitet, welcher in einem temperierten Bad sterilisierten Wassers angeordnet ist. Mittels einer gesonderten Kühleinheit kann das Wasser auf einem vorgegebenen Temperaturniveau gehalten werden, wozu u.a. eine mit einem Motor versehene Rührvorrichtung zum Durchmischen des Wassers vorgesehen ist.

Weiterhin ist aus der WO 2005/117546 A2 eine Vorrichtung zur extrakorporalen Kühlung bekannt. Auch diese Vorrichtung weist eine den Blutfluss fördernde Pumpe sowie eine Einweg-Wärmetauscherkassette auf. Die Kassette ist dabei in einem primären, als Verdampfer ausgebildeten Wärmetauscher angeordnet. Der primäre Wärmetauscher wird dabei mittels eines Kompressors und eines Kondensators aktiv gekühlt.

Die WO 2006/116603 A2 beschreibt ferner eine Vorrichtung und ein Verfahren zur Temperaturmodifikation eines Patienten. Dies wird mittels eines Wärmetauscherkatheters erreicht, in welchem ein Wärmetauschermedium zirkuliert.

Andere Verfahren zur Körperkühlung erfolgen unter großem apparativem Aufwand, so zum Beispiel unter Verwendung einer Herz-Lungen-Maschine oder eines Hämodialyseapparats. Mittels derartiger Geräte kann zwar eine Kühlung des Bluts und somit des Körpers erfolgen. Jedoch sind diese Vorrichtungen eigentlich für andere medizinische Einsatzzwecke konzipiert und stehen allein schon aufgrund ihrer vergleichsweise hohen Anschaffungskosten im ambulanten Bereich oder in Notaufnahmen von Krankenhäusern nur bedingt zur Verfügung.

Es ist daher Ziel der vorliegenden Erfindung, eine apparativ vereinfachte, leicht zu bedienende und kostengünstig zu realisierende Vorrichtung zur Regulierung der Körpertemperatur bereitzustellen, die einen breiten Einsatz der Körperkühlung, insbesondere im ambulanten und mobilen Bereich, vor allem in der Notfallmedizin ermöglicht. Die Vorrichtung soll darüber hinaus möglichst einfach an einen thermisch zu behandelnden Patienten anschließbar sein und höchste Sicherheitsanforderungen im Hinblick auf die Gesundheit des Patienten erfüllen.

Die der Erfindung zugrundeliegende Aufgabe wird mittels einer Vorrichtung zur extrakorporalen Regulierung einer Körperflüssigkeit gemäß Patentanspruch 1 gelöst wobei vorteilhafte Ausgestaltungen der Erfindung Gegenstand abhängiger Patentansprüche sind.

Die erfindungsgemäße Vorrichtung ist für die extrakorporale Regulierung, insbesondere zur Kühlung, aber auch zur Erwärmung einer Körperflüssigkeit, insbesondere von Blut ausgebildet. Sie weist einen extrakorporalen, das heißt außerhalb des zu behandelnden Körpers vorzusehenden Wärmetauscher auf, der einerseits von der zu kühlenden Körperflüssigkeit, das heißt von Blut und andererseits von einem Kühlmedium durchströmbar ist. Das Kühlmedium und die Körperflüssigkeit sind innerhalb des Wärmetauschers hermetisch voneinander getrennt und stehen über zumindest ein Wärmetauscherrohr in thermischem Kontakt miteinander.

Des Weiteren ist am oder im Wärmetauscher zumindest ein Temperatursensor vorgesehen, der zur Erfassung der Temperatur der den Wärmetauscher durchströmenden Körperflüssigkeit dient. Weiterhin ist eine mit dem Temperatursensor und/oder mit dem Wärmetauscher gekoppelte Steuereinheit vorgesehen. Diese ist dazu ausgebildet, in Abhängigkeit einer vom Temperatursensor ermittelten Temperatur der Körperflüssigkeit eine Durchflussmenge des Kühlmediums durch den Wärmetauscher zu regeln. Auf diese Art und Weise kann mittels Durchflussregulierung des Kühlmediums die Temperatur der den Wärmetauscher durchströmenden Körperflüssigkeit auf einen vorgegebenen Soll-Wert eingestellt werden.

Die Steuereinheit bildet mit einer Durchflussmengenregelung und dem Temperatursensor einen Regelkreis zum Einstellen der Temperatur der Körperflüssigkeit auf einen vorgegebenen Soll-Wert.

Es ist ferner vorgesehen, dass das zumindest eine Wärmetauscherrohr vom Kühlmedium durchströmbar und innerhalb des Wärmetauschers von Körperflüssigkeit umströmbar ist. Alternativ kann aber auch eine umgekehrte Anordnung vorgesehen werden, bei welcher die thermisch zu behandelnde Körperflüssigkeit durch das zumindest eine Wärmetauscherrohr strömt und dieses Wärmetauscherrohr vom Kühlmedium umströmt oder umspült wird.

Nach einer bevorzugten Ausgestaltung weist der Wärmetauscher an einem Zulauf und/oder an einem Ablauf für die Körperflüssigkeit einen Temperatursensor zur Ermittlung der jeweiligen Zulauf- bzw. Ablauftemperatur auf. Anhand der Differenz zwischen Zulauf- und Ablauftemperatur kann die Durchflussmenge des Kühlmediums mittels der Steuereinheit präzise geregelt werden. Wenn ferner noch die Durchflussmenge der Körperflüssigkeit durch den Wärmetauscher mittels einer weiteren Sensoreinrichtung bestimmt und der Steuereinheit zugeführt wird, kann unter Kenntnis der physikalischen bzw. thermodynamischen Parameter des Wärmetauschers, der thermisch zu behandelnden Körperflüssigkeit und des Kühlmediums die der Körperflüssigkeit zuzuführende oder zu entziehende Wärmemenge vergleichsweise präzise berechnet und dementsprechend die Durchflussmenge des auf einem vorgegebenen Temperaturniveau befindlichen Kühlmediums bestimmt und dem Wärmetauscher zugeführt werden.

Nach einer weiteren bevorzugten Ausgestaltung sind im Wärmetauscher mehrere, bevorzugt parallel verzweigte Wärmetauscherrohre oder -Leitungen vorgesehen, deren Durchfluss mittels der Steuereinheit jeweils separat und unabhängig voneinander regelbar ist. Die einzelnen Wärmetauscherrohre können hierbei parallel zueinander, spiralartig gedreht, ineinander geschachtelt sowie mäanderartig, bzw. nahezu beliebig im von der Körperflüssigkeit durchströmbaren Innenraum des Wärmetauschers verlaufen. Je nach gefordertem Grad des Wärmeaustauschs können einzelne Wärmetauscherrohre in ihrem Durchfluss gedrosselt oder gar gänzlich strömungstechnisch unterbrochen werden.

Es erweist sich ferner von Vorteil, wenn die einzelnen Wärmetauscherrohre jeweils separat oder gebündelt an die Steuereinheit oder an ein von der Steuereinheit gesteuertes Regelmodul ankoppelbar sind. Auf diese Art und Weise kann die Steuereinheit mittels einzelner ansteuerbarer Ventile und Stellmotoren die Durchflussmengen in den einzelnen Wärmetauscherrohren entsprechend der geforderten Kühl- oder Heizleistung verändern.

Nach einer bevorzugten Ausgestaltung ist ferner ein thermisch isoliertes Reservoir zur Aufnahme und/oder temperierten Lagerung einer vorbestimmten Menge des Kühlmediums vorgesehen. Das Reservoir kann insbesondere bei einer Anwendung der Kühlvorrichtung im ambulanten oder mobilen Einsatzbereich zum Beispiel in einem Kühlschrank oder dergleichen Kühleinrichtungen gelagert werden. Ein aktives Abkühlen des Kühlmediums ist insoweit nicht erforderlich, sondern es kann auf ein bereitgestelltes thermisches Reservoir zurückgegriffen werden.

Es ist dabei insbesondere von Vorteil, wenn das Reservoir, die Steuereinheit und/oder der Wärmetauscher jeweils miteinander korrespondierende fluidverbindende Kupplungen zur Bildung eines regelbaren Stroms des Kühlmediums durch den Wärmetauscher aufweisen. So kann insbesondere vorgesehen werden, den Wärmetauscher kühlmitteleinlassseitig mit dem Reservoir zu koppeln, die Steuereinheit bzw. ihr Regelmodul zwischen Reservoir und Wärmetauscher zu schalten oder aber sogar auslassseitig des Wärmetauschers vorzusehen. Es ist ferner denkbar, das aus dem Wärmetauscher austretende, etwa durch die Körperflüssigkeit erwärmte Kühlmittel in das Reservoir zurückzuführen, wobei Ablauf und Zulauf des Reservoirs bevorzugt hermetisch voneinander zu trennen sind.

Das bevorzugt thermisch isolierte Reservoir kann beispielsweise zwei oder mehrere Behältnisse aufweisen, von denen zumindest eines im Auslieferungszustand mit dem Kühlmedium gefüllt, ein anderes jedoch leer ist und daher zur Aufnahme des durch den Wärmetauscher geströmten Kühlmittels dienen kann. Indem genormte fluidverbindende Kupplungen am Wärmetauscher, an der Steuereinheit bzw. an ihrem Regelmodul und am Reservoir für das Kühlmedium vorgesehen sind, kann im Verlauf des Kühlprozesses ein geleertes Behältnis oder Reservoir durch ein gefülltes Reservoir ausgetauscht werden. Sofern das Reservoir mit einem Rücklaufbehältnis versehen ist, kann es nach Gebrauch einer externen Kühleinrichtung, wie etwa einem Kühlschrank, zugeführt werden und unter Umständen für eine weitere Kühlung Verwendung finden.

Alternativ zu dem beschriebenen geschlossenen Kühlkreislauf ist ferner denkbar, den Ablauf des Wärmetauschers offen auszugestalten und zugeführtes Kühlmedium am Wärmetauscherauslass nicht zurückzuführen, sondern dieses an die Umgebung abzugeben. Auf diese Art und Weise kann ein vergleichsweise einfacher, offener Kühlkreislauf realisiert werden. Die Steuereinheit ist ferner dazu ausgebildet, auch unterschiedliche Temperaturniveaus des Kühlmediums zu berücksichtigen.

In dem eine Regelung der Kühlleistung der erfindungsgemäßen Vorrichtung ausschließlich über die Regulierung der Durchflussmenge des Kühlmediums durch den Wärmetauscher bereitgestellt werden kann, eröffnet die erfindungsgemäße Vorrichtung völlig neuartige apparative Ansätze zur Bereitstellung einer extrakorporalen Kühlung, insbesondere im Bereich der Notfallmedizin. So kann durch die Regulierung des Durchsatzes oder des Durchflusses des bereitgestellten Kühlmediums ein offener, d.h. nicht geschlossener Kühlkreislauf bereitgestellt werden, wobei auf ein Reservoir vorgekühlten Kühlmediums zurückgegriffen werden kann.

Das Kühlmedium kann beispielsweise in Form von Beuteln oder dgl. Behältnissen in einen Kühlschrank oder einer vergleichbaren Kühleinrichtung gelagert und bei Bedarf aus der gekühlten Umgebung entnommen sowie schließlich für die extrakorporale Kühlung und Herbeiführung der Hypothermie mit dem Wärmetauscher für die Körperflüssigkeit thermisch verbunden werden. Sobald ein mit Kühlflüssigkeit gefüllter Behälter während der Kühlprozedur geleert wurde, kann er durch einen weiteren, ebenfalls vorgekühlten Behälter ersetzt werden.

Durch die Regulierung des Durchsatzes bzw. der Durchflussmenge des Kühlmediums durch den Wärmetauscher kann ferner in besonders einfacher Art und Weise auch auf Temperaturänderungen des Kühlmediums reagiert werden. Da während eines Herunterkühlens eines Patienten auf eine vorgegebene Temperatur eine vergleichsweise große Menge an Kühlmedium benötigt wird, kann für die Aufrechterhaltung einer bereits abgekühlten Körpertemperatur die Durchflussmenge entsprechend reduziert werden.

Sollte sich während der Hypothermie-Anwendung die Temperatur des Kühlmediums selbst ändern, kann dieser Änderung, insbesondere einer zumindest geringfügigen Erwärmung des Kühlmediums durch eine Steigerung der Durchflussmenge Rechnung getragen werden. Die Gesamtkühlleistung, die über den Wärmetauscher an die Körperflüssigkeit und somit an den zu kühlenden Körper abzugeben ist, kann auf diese Art und Weise annähernd konstant gehalten werden.

Die Regulierung der Durchflussmenge des bereitgestellten Kühlmediums kann den apparativen Aufwand deutlich verringern. Etwaige Kompressoren, Verdampfer oder Kondensatoren, wie sie in gängigen Kühlmittelkreisläufen vorzuhalten sind, werden vorliegend nicht benötigt. Ferner kann auch der Zustrom bzw. der Durchfluss des Kühlmediums durch den Wärmetauscher weitgehend pumpenlos und rein schwerkraftbedingt erfolgen.

Wenngleich der Einsatz der erfindungsgemäßen Vorrichtung rein für Kühlzwecke beschrieben ist, kann sie auch gleichermaßen zur Erwärmung von Körperflüssigkeiten, insbesondere von Blut dienen. Anstelle eines kalten oder vorgekühlten Kühlmediums wäre lediglich ein entsprechend temperiertes Kühlmedium zu verwenden.

Zur Erzielung eines Soll-Werts der Körperflüssigkeitstemperatur ist bei einem geringen Temperaturunterschied zwischen Kühlmedium und der dem Wärmetauscher zugeführten Körperflüssigkeit eine vergleichsweise hohe Durchflussmenge einzustellen, während bei einer hohen Temperaturdifferenz zwischen zugeführter Körperflüssigkeit und dem Kühlmedium eine weitaus geringere Durchflussmenge des Kühlmediums zur thermischen Behandlung der Körperflüssigkeit und somit zur Abkühlung bzw. zur Erwärmung des jeweiligen Patienten vorzusehen ist.

Nach einem weiteren vorteilhaften Aspekt ist als Kühlmedium sterile Kochsalzlösung, vorzugsweise 0,9 prozentige oder isotonische Kochsalzlösung vorgesehen, sodass bei einer Leckage des die Körperflüssigkeit führenden Kreislaufs keine Kontamination oder gar gesundheitliche Schädigung des Patienten auftreten kann. Zudem ist die Verwendung steriler Kochsalzlösung als Kühlmedium äußerst preiswert, sodass eine Rückführung des den Wärmetauscher durchströmten Kühlmittels aus Kostengründen generell nicht erforderlich ist.

In einer weiteren Ausgestaltung der Erfindung ist zumindest ein Drucksensor und/oder ein Gassensor im Wärmetauscher vorgesehen, der oder die mit der Steuereinheit gekoppelt sind und von dieser ausgewertet werden. Mittels des Drucksensors kann eine Verstopfung des Wärmetauschers, welche zum Beispiel bei einer Bildung von Thromben auftreten kann, detektiert werden.

Auslassseitig am Wärmetauscher ist zudem ein Filter vorgesehen, der eine Verunreinigung der dem Körper zurückzuführenden Körperflüssigkeit verhindern soll. Hierbei kann zum Beispiel ein 40 µm-Filter Anwendung finden.

Mittels des Gassensors können Gasblasen im Wärmetauscher bzw. Wärmetauscherkreislauf detektiert werden. Infolge einer Gasblasendetektion ist hierbei das Auslösen einer Alarmfunktion vorgesehen, mittels derer vor Gasblasen in der zirkulierenden Körperflüssigkeit bzw. im Blutkreislauf entsprechend gewarnt werden kann.

Nach einer weiteren bevorzugten Ausgestaltung ist vorgesehen, dass die Vorrichtung zur extrakorporalen Kühlung zumindest im die Körperflüssigkeit führenden Leitungs- oder Kreislaufsystem pumpenlos ausgebildet ist. Eine Strömung bzw. ein Durchströmen des Wärmetauschers mit der zu kühlenden bzw. zu erwärmenden Körperflüssigkeit kann durch Kopplung des Wärmetauschereinlasses mit einem arteriellen System und Kopplung des Wärmetauscherauslasses mit dem venösen System eines Herz-Kreislauf-Systems eines Patienten erreicht werden.

Das arteriell-venöse Druckgefälle ist grundsätzlich ausreichend, um einen für Kühlzwecke erforderlichen Blutfluss durch den Wärmetauscher zu erreichen. Die arteriell-venöse Brücke kann hierbei unter Verwendung bekannter Kanülen, insbesondere mittels eines zum Beispiel bei der Hämodialyse verwendeten Shunts erreicht werden. Bei einer venös-venösen Anbindung des Wärmetauschers an den zu kühlenden Körper ist die extrakorporale Kühlvorrichtung mit einer Pumpe, etwa einer Rollen- oder Fingerpumpe zu versehen.

Unabhängig von der strömungstechnischen Anbindung des Wärmetauschers an den Patienten kann der Durchfluss des Kühlmediums durch den Wärmetauscher ebenfalls mittels einer Pumpe erfolgen. Alternativ ist insbesondere bei einem offen ausgebildeten Kühlkreislauf aber auch denkbar, ein Durchströmen des Wärmetauschers mit dem Kühlmedium rein schwerkraftbedingt zu implementieren, indem zum Beispiel der das Kühlmedium enthaltende Behälter auf einem Höhenniveau oberhalb des Wärmetauschers angeordnet wird.

Kühlmittelstrom und die Strömung der Körperflüssigkeit im Wärmetauscher sind in Gegenrichtung ausgebildet, um einen möglichst effektiven Wärmeaustausch zwischen den Flüssigkeiten zu erzielen. Ferner ist vorgesehen, auch die Zu- bzw. Ablauftemperatur des Kühlmediums zum bzw. vom Wärmetauscher mittels geeigneter Sensoren zu bestimmen und auch diese Temperaturinformationen der Steuereinheit zuzuführen. Auf diese Art und Weise kann der Wärmeaustauschgrad der Vorrichtung in der Steuereinheit berechnet und dementsprechend der Durchfluss des Kühlmediums durch den Wärmetauscher präzise reguliert werden.

In einer weiteren bevorzugten Ausgestaltung ist vorgesehen, dass der gesamte Wärmetauscher, insbesondere auch sein von Körperflüssigkeit zu durchströmendes Volumen mit steriler isotonischer ggf. gekühlter Kochsalzlösung vorgefüllt ist. So kann der Wärmetauscher selbst unmittelbar durch Anschließen an das Herz-Kreislauf-System eines Patienten bereits einen gewissen Kühleffekt herbeiführen. Zudem dient die Vorab-Befüllung des Wärmetauschers einem problemlosen und luftfreien Anschluss an ein Blutkreislaufsystem. Weiterhin kann mittels der Vorfüllung bei einem arteriell-venösen Anschluss an einen Patienten unmittelbar zu Beginn des einsetzenden Blutflusses durch den Wärmetauscher dessen Volumenstrom bestimmt werden.

So kann bei einem vorgekühlten und vorgefüllten Wärmetauscher aus der Zeitdauer zwischen dem Beginn einer Blutströmung durch den Wärmetauscher bis zum Erreichen einer Maximaltemperatur am Wärmetauscherauslass der Durchfluss der Körperflüssigkeit durch den Wärmetauscher bestimmt werden. Die Steuereinheit kann diese Zeitdauer selbsttätig ermitteln und hieraus zum Beispiel die Durchflussmenge der Körperflüssigkeit durch den Wärmetauscher berechnen, bevor einzelne Wärmetauscherrohre zusätzlich mit dem vorgekühlten Kühlmedium beaufschlagt werden.

Zur Vermeidung der Bildung von Thromben ist ferner vorgesehen, sämtliche körperflüssigkeitsführenden Leitungen der Kühlvorrichtung mit einer Beschichtung zu versehen, die insbesondere einer Trombozytenaggregation entgegenwirkt.

Weitere Ziele, Merkmale sowie vorteilhafte Anwendungsmöglichkeiten der Erfindung werden in der nachfolgenden Beschreibung unter Bezugnahme auf Ausführungsbeispiele erläutert, wobei sämtliche im Text beschriebenen als auch in den Figuren bildlich dargestellten Merkmale sowohl in Alleinstellung als auch in jeglicher sinnvollen Kombination untereinander den Gegenstand der Erfindung bilden.

Es zeigen:
- Fig. 1: eine schematische isolierte Darstellung eines Wärmetauschers,
- Fig. 2: eine schematische Darstellung eines fluidführenden verzweigenden Leitungsstücks,
- Fig. 3: eine schematische Darstellung eines thermisch isolierten Reservoirs für das Kühlmedium und
- Fig. 4: eine schematische Darstellung der Steuereinheit.

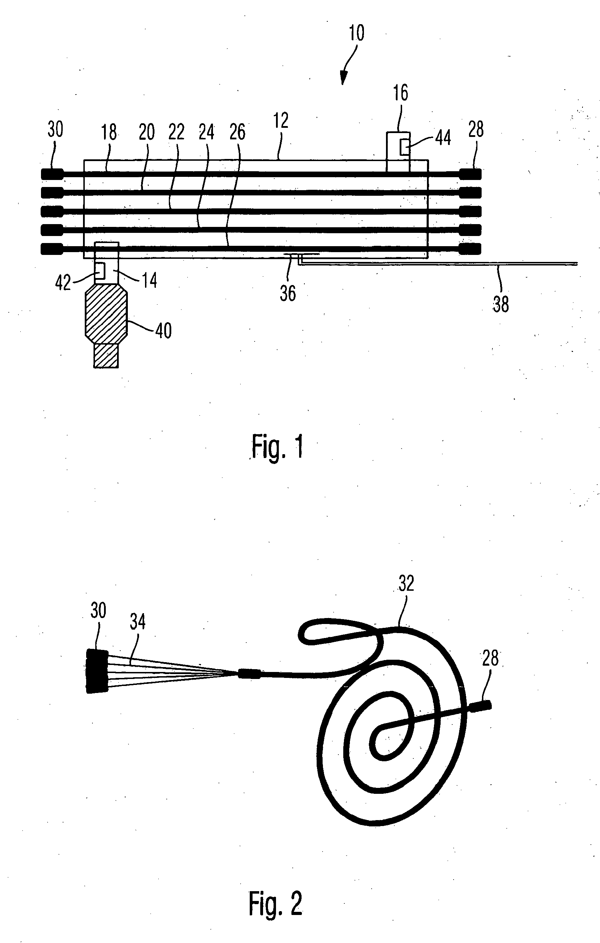

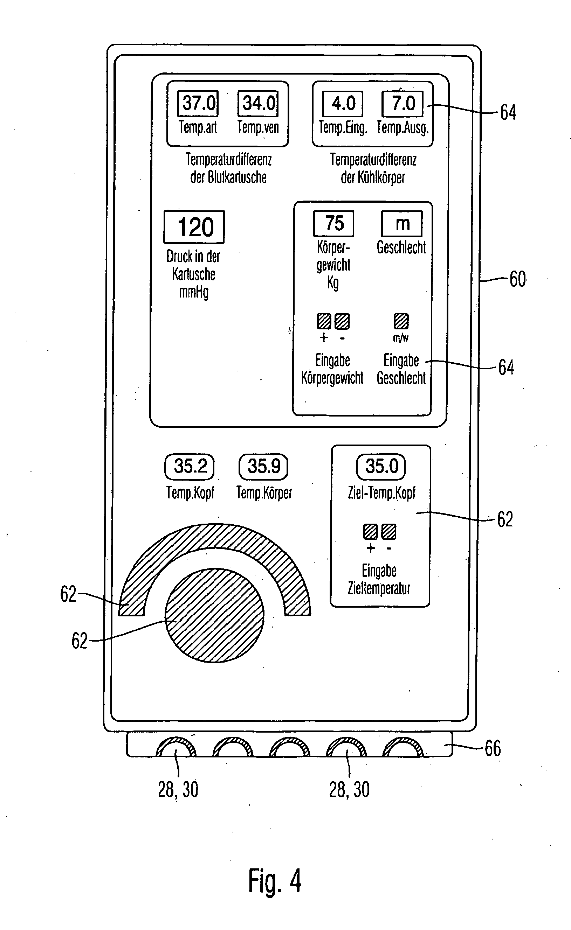

Der in Fig. 1 gezeigte Wärmetauscher 10 weist ein Gehäuse 12 mit einem Zulauf 16 und einem Ablauf 14 für die Körperflüssigkeit auf. Über den Zulauf 16, der zum Beispiel arteriell mit dem Herz-Kreislauf-System eines Patienten verbunden werden kann, wird zum Beispiel Blut in das frei durchströmbare Innenvolumen des Gehäuses 12 geleitet, welches über den Auslass 14 und den nachgeschalteten Filter 40 wieder dem zu kühlenden oder auch zu erwärmenden Körper venös zugeführt wird. Im Bereich der Zu- und Abläufe 16, 14 sind Temperatursensoren 44, 42 vorgesehen, die mit der in Fig. 4 isoliert dargestellten Steuereinrichtung 60 gekoppelt sind.

Der Wärmetauscher 10 weist ferner eine Reihe von Wärmetauscherrohren 18, 20, 22, 24, 26 auf, die in der in Fig. 1 gezeigten Darstellung das Gehäuse 12 des Wärmetauschers 10 parallel zueinander verlaufend durchsetzen. Abweichende Geometrien der einzelnen Rohre 18, 20, 22, 24, 26, etwa spiral- oder mäanderartig sind jedoch gleichermaßen denkbar. Die einzelnen Wärmetauscherrohre 18, 20, 22, 24, 26 sind an ihren Enden jeweils mit miteinander korrespondierenden, bevorzugt genormten, etwa mit Luer-Lock Kupplungsstücken 28, 30 versehen, die ein universelles Ankoppeln zum Beispiel an eine in Fig. 2 isoliert dargestellte Schlauchverbindung ermöglichen.

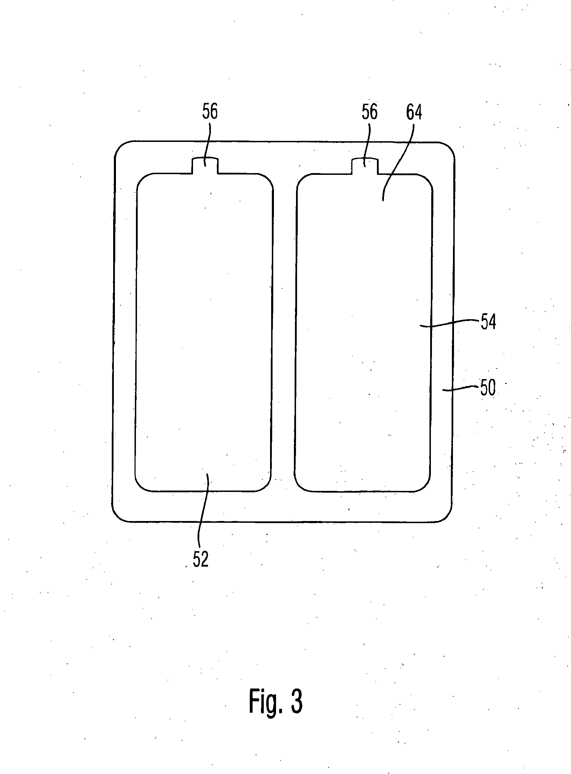

Die in Fig. 2 gezeigte Verbindung weist ein Schlauchstück 32 auf, welches sich einen Ends in fünf einzelne Zweige 34 aufteilt, die jeweils mit einem Kupplungsstück 30 versehen sind. Diese Zweige 34 sind zum Beispiel an die in Fig. 1 rechts dargestellten Kupplungsstücke 28 der einzelnen Wärmetauscherrohre 18, 20, 22, 24, 26 ankoppelbar. Andern Ends kann das Schlauchstück 32 etwa mittels einer Kupplung 28 an das in Fig. 3 gezeigte thermisch isolierte Reservoir 50, insbesondere an einen der darin befindlichen Behälter 52, 54, so z.B. an deren Gegenkupplung 56 angeschlossen werden.

Bei einem geschlossenen System ist zum Beispiel vorgesehen, einen vorgekühlten und mit Kühlmedium, insbesondere mit isotonischer steriler Kochsalzlösung gefüllten Behälter 52 über das Schlauch- und Verbindungsstück 32 mit den einzelnen Wärmetauscherrohren 18, 20, 22, 24, 26 fluidführend zu koppeln. Andern Ends können die Wärmetauscherrohre 18, 20, 22, 24, 26 mit einem Regelmodul 66 des Steuergeräts gekoppelt werden, wie dies in Fig. 4 isoliert dargestellt ist. Die einzelnen Kupplungsanschlüsse 28, 30 des Regelmoduls 66 des Steuergeräts dienen einer separaten und unabhängigen Regelung der Durchflussmengen durch die einzelnen Wärmetauscherrohre 18, 20, 22, 24, 26, um die Kühl- bzw. Heizleistung des Wärmetauschers 10 gezielt regeln zu können.

Stromabwärts desyRegelmoduls 60 können die fünf Einzelleitungen etwa mit einem weiteren Verbindungsstück gemäß Fig. 2 fluidführend vereinigt und mit einem leeren Behälter 54 des Reservoirs 50 gekoppelt werden. Ein derartiges System erfordert typischerweise den Einsatz einer das Kühlmedium befördernden, in den Figuren nicht näher dargestellten Pumpe, die ggf. in das Steuergerät 60 oder in dessen Regelmodul 66 integriert sein kann.

Anstelle eines in Fig. 3 angedeuteten geschlossenen Kühlkreislaufs kann auch ein geöffneter Kühlkreislauf implementiert werden, wobei auslassseitig der Wärmetauscherrohre 18, 20, 22, 24, 26 des Wärmetauschers 10 das dem Wärmetauscher 10 zugeführte Kühlmedium an die Umgebung abgegeben werden kann.

Die Anordnung des Steuergeräts oder der Steuereinheit 60 bzw. ihres Regelmoduls 66 kann stromaufwärts als auch stromabwärts der einzelnen Wärmetauscherrohre 18, 20, 22, 24, 26 des Wärmetauschers 10 erfolgen. In Fig. 1 ist ferner ein im von der Körperflüssigkeit durchströmbaren Gehäuse 12 angeordneter Drucksensor 36 gezeigt, der über ein Signalkabel 38 mit der Steuereinheit 60 gekoppelt ist. Ein über einen vorgegebenen Schwellwert ansteigender Druck ist hierbei ein Indiz für eine Verstopfung des Wärmetauschers 10.

Die in Fig. 4 schematisch dargestellte Steuereinheit 60 weist verschiedene Eingabemittel 62 sowie verschiedene Anzeigemittel 64 auf. Sämtliche Temperatur-, Druck- und Durchflussparameter betreffend Zu- und Ablauf des Kühlmediums als auch Zu- und Ablauf der Körperflüssigkeit in bzw. aus dem Wärmetauscher 10 können zur Berechnung einer zur Verfügung zu stellenden Kühlleistung der Steuereinheit 60 zugeführt werden. So kann zum Beispiel ein Soll-Wert für die Körperflüssigkeit an der Steuereinheit 60 eingestellt werden. Unter Angabe weiterer patientenrelevanter Parameter, wie zum Beispiel des Geschlechts und des Körpergewichts sowie unter Berücksichtigung der Zu- und Ablauftemperaturen sowohl von Körperflüssigkeit und Kühlmedium am Wärmetauscher und des Fluiddrucks der Körperflüssigkeit sowie ggf. des die Wärmetauscherrohre 18, 20, 22, 24, 26 durchströmenden Kühlmediums, insbesondere durch Differenzenbildung und unter Berücksichtigung bekannter oder empirisch zu bestimmender fluidspezifischer Wärmekoeffizienten kann die Steuereinheit 60 den Gesamtdurchfluss durch die einzelnen Wärmetauscherrohre 18, 20, 22, 24, 26 zur schnellstmöglichen Erzielung einer Soll-Körperflüssigkeitstemperatur berechnen und dementsprechend die Wärmetauscherrohre 18, 20, 22, 24, 26 mit Hilfe des Regelmoduls 66 mit dem Kühlmedium beaufschlagen.

Die einzelnen, in den Fig. 1 bis 4 jeweils separat dargestellten Komponenten können als einzelne Module der erfindungsgemäßen Kühlvorrichtung kommerziell hergestellt und vertrieben werden und auf den jeweiligen Anwendungsfall universell in vielfältigster Art und Weise miteinander gekoppelt werden. Unter Umständen ist sogar das Hintereinanderschalten mehrerer Wärmetauscher 10 sowie ein während des Kühlprozesses stattfindendes Austauschen etwa verbrauchter Reservoire 50 an Kühlmedium möglich. Insgesamt kann die für die extrakorporale Kühlvorrichtung zur Erzielung einer milden Hypertonie besonders preiswert und in einer handlichen sowie transportablen Bauform implementiert werden, sodass sie sich bestens für den Einsatz in der Notfallmedizin eignet.

### Bezugszeichenliste

- 10: Wärmetauscher
- 12: Wärmetauschergehäuse
- 14: Zulauf
- 16: Ablauf
- 18: Wärmetauscherrohr
- 20: Wärmetauscherrohr
- 22: Wärmetauscherrohr
- 24: Wärmetauscherrohr
- 26: Wärmetauscherrohr
- 28: Kupplung
- 30: Kupplung
- 32: Verbindungsstück
- 34: Verzweigung
- 36: Drucksensor
- 38: Signalleitung
- 40: Filter
- 42: Temperatursensor
- 44: Temperatursensor
- 50: Reservoir
- 52: Behälter
- 54: Behälter
- 56: Kupplung
- 60: Steuereinrichtung
- 62: Eingabemittel
- 64: Display/Ausgabemittel
- 66: Regelmodul

## Patentansprüche

1. Vorrichtung zur extrakorporalen Regulierung der Temperatur einer Körperflüssigkeit mit:
- einem Wärmetauscher (10), welcher von der zu kühlenden Körperflüssigkeit und von einem Kühlmedium durchströmbar ist, wobei das Kühlmedium und die Körperflüssigkeit hermetisch voneinander getrennt und über zumindest ein Wärmetauscherrohr (18, 20, 22, 24, 26) in thermischem Kontakt miteinander stehen,
- zumindest einem Temperatursensor (42, 44) zur Erfassung der Temperatur der den Wärmetauscher (10) durchströmenden Körperflüssigkeit und mit
- einer Steuereinheit (60), welche dazu ausgebildet ist, in Abhängigkeit einer ermittelten Temperatur der Körperflüssigkeit eine Durchflussmenge des Kühlmediums durch den Wärmetauscher (10) zu regeln, wobei das zumindest eine Wärmetauscherrohr (18, 20, 22, 24, 26) vom Kühlmedium durchströmbar und innerhalb des Wärmetauschers (10) von Körperflüssigkeit umströmbar ist oder wobei das zumindest eine Wärmetauscherrohr (18, 20, 22, 24, 26) von Körperflüssigkeit durchströmbar und innerhalb des Wärmetauschers (10) vom Kühlmedium umströmbar ist.

2. Vorrichtung nach Anspruch 1, wobei der Wärmetauscher (10) an einem Zulauf und/oder an einem Ablauf für die Körperflüssigkeit einen Temperatursensor (42, 44) zur Ermittlung der jeweiligen Zulauf- bzw. Ablauftemperatur aufweist.

3. Vorrichtung nach einem der vorhergehenden Ansprüche, wobei im Wärmetauscher (10) mehrere parallel verzweigte Wärmetauscherohre (18, 20, 22, 24, 26) vorgesehen sind, deren Durchfluss mittels der Steuereinheit (60) jeweils separat und unabhängig voneinander regelbar ist.

4. Vorrichtung nach Anspruch 3, wobei die einzelnen Wärmetauscherrohre (18, 20, 22, 24, 26) an die Steuereinheit (60) oder an ein von der Steuereinheit gesteuertes Regelmodul (66) ankoppelbar sind.

5. Vorrichtung nach einem der vorhergehenden Ansprüche, mit zumindest einem thermisch isolierten Reservoir (50) zur Aufnahme und/oder temperierten Lagerung einer vorbestimmten Menge des Kühlmediums.

6. Vorrichtung nach Anspruch 5, wobei das Reservoir (50), die Steuereinheit (60) und/oder der Wärmetauscher (10) jeweils miteinander korrespondierende fluidverbindende Kupplungen (28, 30, 56) zur Bildung eines regelbaren Stroms des Kühlmediums durch den Wärmetauscher (10) aufweisen.

7. Vorrichtung nach einem der vorhergehenden Ansprüche, wobei als Kühlmedium sterile Kochsalzlösung vorgesehen ist.

8. Vorrichtung nach einem der vorhergehenden Ansprüche, wobei ein Drucksensor und/oder ein Gassensor zur Bestimmung des Körperflüssigkeitsdrucks bzw. zur Detektion von Gasblasen im Wärmetauscher (10) vorgesehen ist.

9. Vorrichtung nach einem der vorhergehenden Ansprüche, wobei der Ablauf des Wärmetauschers (10) mit dem venösen System und der Zulauf für den Wärmetauscher (10) mit dem arteriellen oder venösen System eines Herz-Kreislaufsystems koppelbar ist.

## Claims

1. Device for extracorporeal regulation of the temperature of a body fluid, with:
- a heat exchanger (10) through which the body fluid to be cooled and a coolant can flow, wherein the coolant and the body fluid are hermetically separate from each other and are in thermal contact with each other via at least one heat exchanger pipe (18, 20, 22, 24, 26),
- at least one temperature sensor (42, 44) for detecting the temperature of the body fluid flowing through the heat exchanger (10), and with
- a control unit (60) designed to regulate a flow rate of the coolant through the heat exchanger (10) depending on a determined temperature of the body fluid, wherein the coolant can flow through the at least one heat exchanger pipe (18, 20, 22, 24, 26) and body fluid can flow around it inside the heat exchanger (10), or wherein body fluid can flow through the at least one heat exchanger pipe (18, 20, 22, 24, 26) and the coolant can flow around it inside the heat exchanger (10).

2. Device according to Claim 1, wherein the heat exchanger (10) has, at an inlet and/or an outlet for the body fluid, a temperature sensor (42, 44) for determining the respective inlet and/or outlet temperature.

3. Device according to one of the preceding claims, wherein several heat exchanger pipes (18, 20, 22, 24, 26) which are branched in parallel are provided in the heat exchanger (10), which heat exchanger pipes (18, 20, 22, 24, 26) can be regulated separately and independently of each other, in terms of the flow passing through them, by means of the control unit (60) .

4. Device according to Claim 3, wherein the individual heat exchanger pipes (18, 20, 22, 24, 26) can be coupled to the control unit (60) or to a regulator module (66) controlled by the control unit.

5. Device according to one of the preceding claims, with at least one thermally insulated reservoir (50) in which a predetermined amount of the coolant is received and/or is stored at a controlled temperature.

6. Device according to Claim 5, wherein the reservoir (50), the control unit (60) and/or the heat exchanger (10) each have fluid-connecting couplings (28, 30, 56) that correspond to one another in order to produce a regulatable stream of the coolant through the heat exchanger (10).

7. Device according to one of the preceding claims, wherein sterile saline solution is provided as coolant.

8. Device according to one of the preceding claims, wherein a pressure sensor and/or a gas sensor is provided for determining the pressure of the body fluid and for detecting gas bubbles in the heat exchanger (10), respectively.

9. Device according to one of the preceding claims, wherein the outlet of the heat exchanger (10) can be coupled to the venous system and the inlet for the heat exchanger (10) can be coupled to the arterial or venous system of a cardiovascular system.

## Revendications

1. Dispositif de régulation extracorporelle de la température d'un liquide corporel, le dispositif présentant :
un échangeur de chaleur (10) dans lequel peuvent s'écouler le liquide corporel à refroidir et un fluide de refroidissement, le fluide de refroidissement et le liquide corporel étant séparés hermétiquement l'un de l'autre et étant amenés en contact thermique mutuel par au moins un tube (18, 20, 22, 24, 26) d'échangeur de chaleur,
au moins une sonde de température (42, 44) qui saisit la température du liquide corporel qui s'écoule dans l'échangeur de chaleur (10) et
une unité de commande (60) configurée pour réguler en fonction de la température qui a été déterminée pour le fluide corporel le débit de fluide de refroidissement s'écoulant dans l'échangeur de chaleur (10),
le fluide de refroidissement pouvant s'écouler dans le ou les tubes (18, 20, 22, 24, 26) d'échangeur de chaleur et le fluide corporel pouvant balayer ce ou ces tubes à l'intérieur de l'échangeur de chaleur (10) ou
le liquide corporel pouvant s'écouler dans le ou les tubes (18, 20, 22, 24, 26) d'échangeur de chaleur et ce ou ces tubes pouvant être balayés par le fluide de refroidissement à l'intérieur de l'échangeur de chaleur (10).

2. Dispositif selon la revendication 1, dans lequel l'échangeur de chaleur (10) présente à l'entrée et/ou à la sortie du liquide corporel une sonde de température (42, 44) qui détermine la température d'entrée ou la température de sortie.

3. Dispositif selon l'une des revendications précédentes, dans lequel plusieurs tubes (18, 20, 22, 24, 26) d'échangeur de chaleur ramifiés et parallèles sont prévus dans l'échangeur de chaleur (10), leur débit pouvant être régulé séparément et en fonction les uns des autres au moyen de l'unité de commande (60).

4. Dispositif selon la revendication 3, dans lequel les différents tubes (18, 20, 22, 24, 26) d'échangeur de chaleur peuvent être raccordés à l'unité de commande (60) ou à un module de régulation (66) commandé par l'unité de commande.

5. Dispositif selon l'une des revendications précédentes, présentant au moins un réservoir (50) isolé thermiquement, qui reprend ou conserve à une température contrôlée une quantité prédéterminée de fluide de refroidissement.

6. Dispositif selon la revendication 5, dans lequel le réservoir (50), l'unité de commande (60) et/ou l'échangeur de chaleur (10) présentent chacun des raccords (28, 30, 56) mutuellement correspondants de raccordement de fluide pour former un écoulement régulable de fluide de refroidissement dans l'échangeur de chaleur (10).

7. Dispositif selon l'une des revendications précédentes, dans lequel une solution stérile de sel de cuisine est prévue comme fluide de refroidissement.

8. Dispositif selon l'une des revendications précédentes, dans lequel un capteur de pression et/ou un capteur de gaz sont prévus pour déterminer la pression du liquide corporel ou pour détecter la présence de bulles de gaz dans l'échangeur de chaleur (10) .

9. Dispositif selon l'une des revendications précédentes, dans lequel la sortie de l'échangeur de chaleur (10) peut être raccordée au système veineux et l'entrée de l'échangeur de chaleur (10) au système artériel ou veineux d'un système de circuit cardiaque.
